(19) Europäisches Patentamt
European Patent Office
Office européen des brevets

(11) **EP 2 863 758 B1**

(12) **EUROPEAN PATENT SPECIFICATION**

(45) Date of publication and mention
of the grant of the patent:
**06.11.2019 Bulletin 2019/45**

(51) Int Cl.:
*C12N 9/96* *(2006.01)*          *A23K 40/10* *(2016.01)*
*A23K 40/20* *(2016.01)*        *A23K 40/30* *(2016.01)*
*A23K 20/105* *(2016.01)*       *A23K 20/158* *(2016.01)*
*A23K 20/163* *(2016.01)*       *A23K 20/189* *(2016.01)*
*A23K 20/22* *(2016.01)*        *A23K 20/28* *(2016.01)*
*C12N 9/16* *(2006.01)*         *C12N 9/98* *(2006.01)*

(21) Application number: **13733173.2**

(22) Date of filing: **14.06.2013**

(86) International application number:
**PCT/US2013/045944**

(87) International publication number:
**WO 2013/192043 (27.12.2013 Gazette 2013/52)**

(54) **SANDWICH GRANULE**

SANDWICHGRANULAT

GRANULÉ INTERCALÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.06.2012   US 201261662254 P**

(43) Date of publication of application:
**29.04.2015   Bulletin 2015/18**

(73) Proprietor: **Danisco US Inc.
Palo Alto, California 94304 (US)**

(72) Inventor: **DALE, Douglas A.
Palo Alto, California 94304 (US)**

(74) Representative: **Mewburn Ellis LLP
City Tower
40 Basinghall Street
London EC2V 5DE (GB)**

(56) References cited:
WO-A1-97/23606          WO-A2-02/34871
US-A1- 2008 031 998     US-A1- 2009 274 795
US-B1- 6 268 329

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

This disclosure is directed towards improved compositions for layered granules containing active agents and methods of making and using.

**BACKGROUND OF THE INVENTION**

The use of active agents, such as enzymes, in foods and animal feed has become a common practice. Enzymes are known to improve digestibility of food and animal feed, reduce anti-nutritional factors in food and animal feed, and improve animal productivity.

Inactivation of enzymes can occur during industrial food and feed processes (such as steam pelleting) by, for example, heat treatment, high pressure, shear stress, and chemical treatment (such as pH, surfactant, and solvents). The inactivation is at least partially reversible if the enzyme reactivates after processing, for example, upon cooling after steam treatment and pelleting; the inactivation is irreversible if the catalytic activity does not resume after processing, for example, upon cooling after steam treatment and pelleting. The irreversible inactivation and reduced activity of an enzyme is generally not desirable in processes such as steam pelleting.

When compared with dry feed mixes, feed pellets have properties that are favored by the industry, such as improved feed quality, decreased pathogens, lower dust levels during manufacture, ease of handling, and more uniform ingredient dosing. Preferred industry pelleting processes utilize steam injection, in a process known as conditioning, which adds moisture and elevates the temperature prior to the pelleting step which forces the steam heated feed ingredients, or conditioned mash, through a die. The pelleting process temperatures may be from about 70°C to 95°C, or higher.

Because of the steam, temperatures, compression forces and chemicals used in pelleting processes, the activity or potency of enzymes are often significantly reduced during processing and subsequent storage. In fact, feed enzymes are often provided to the industry as stabilized liquid products that are sprayed onto feed pellets after the pelleting process to avoid enzyme inactivation. However, homogeneous dosing is difficult to achieve when the enzyme is applied post pelleting, for instance, by spraying the enzyme onto the pellets, and the cost of the equipment to add enzyme post-pelleting is high. Alternatively, liquid enzyme formulations, or dry mix enzyme formulations, may be added to the mixer prior to pelleting. In certain instances, higher levels of enzymes than otherwise needed may be added in order to compensate for losses during pelleting.

There is a need in the food and feed industries for stable, durable enzyme granules to serve as components in formulations that are subjected to steam treatment pelleting processes without appreciable loss of enzyme activity.

Approaches to avoid the problem of irreversibly inactivating enzymes or reducing the activity of the enzyme in industrial processes include identifying new sources of an enzyme (e.g. the identification of a known enzyme in an extreme thermophile microorganism) or identifying means to stabilize known enzymes. Klibanov, 1983, (Stabilization of Enzymes against Thermal Inactivation, Advances in Applied Microbiology, volume 29, page 1-28) discloses that there are three basic means for stabilizing enzymes: (1) immobilization, (2) chemical modification and (3) inclusion of additives. However, Klibanov (1983) further discloses that any one of these methods could lead to stabilization or destabilization, or have no effect at all. While previous formulation approaches have made some progress in this area (see for example WO9854980, WO9739116, WO2007044968), and EP1996028) the present teachings make an additional advance in overcoming some of these problems by use of an improved granule structure.

For ease of reference we have described elements of the present teachings under one or more headings. It is to be noted that the teachings under each of the headings also apply to the teachings under the other headings. For example, each of the stated embodiments and aspects concerning the use of the present teachings is equally an embodiment or aspect concerning the method of the present teachings or the composition of the present teachings. Likewise, each of the stated embodiments and aspects concerning the method or use of the present teachings is equally an embodiment or aspect concerning the composition of the present teachings.

WO 97/23606 describes enzyme granules having a core, an enzyme layer and an outer coating layer in which the enzyme layer, and optimally the core and coating layers, contain a vinyl polymer. US 2008/0031998 describes feed granules comprising a feed enzyme and a zinc salt of an organic acid.

**BRIEF SUMMARY OF THE INVENTION**

In one aspect, the present invention provides a granule comprising;

a core comprising an active agent that comprises an enzyme;

a first moisture barrier layer comprising a moisture barrier material;
a moisture hydrating layer comprising a moisture hydrating material surrounding the first moisture barrier layer; and,
a second moisture barrier layer comprising a moisture barrier material surrounding the moisture hydrating layer;
wherein the moisture barrier material of the first moisture barrier layer comprises PVA and talc, wherein the PVA is fully hydrolysed and present at 2%-4% w/w of the granule, and the talc is present at 5%-7% w/w of the granule.

In a further aspect, the present invention provides a process for producing an animal feed composition comprising:

preparing granules as defined in the claims, the granules having a core comprising an active agent, a first moisture barrier layer, a moisture hydrating layer, and second moisture barrier layer;
mixing the granules together with an unpelleted mixture; and,
pelleting the unpelleted mixture at a temperature of 70°C-95°C.

In further aspects, animal feed pelleted and unpelleted compositions and their uses are provided as set out in the claims.

## BRIEF DESCRIPTION OF THE DRAWINGS

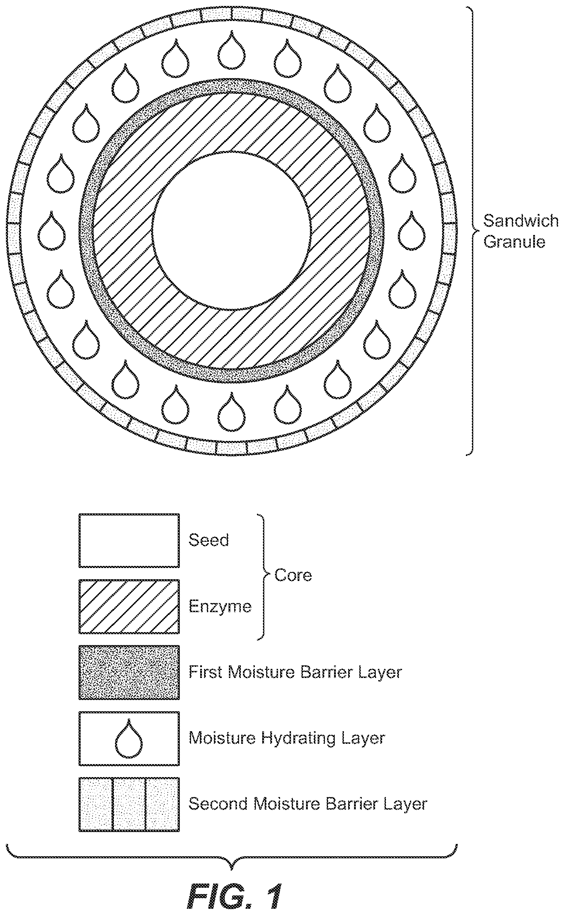

**Figure 1** depicts an illustrative sandwich granule according to the present teachings.

## DETAILED DESCRIPTION

The practice of the present teachings will employ, unless otherwise indicated, conventional techniques of molecular biology (including recombinant techniques), microbiology, cell biology, biochemistry, and animal feed pelleting, which are within the skill of the art. Such techniques are explained fully in the literature, for example, Molecular Cloning: A Laboratory Manual, second edition (Sambrook et al., 1989); Oligonucleotide Synthesis (M. J. Gait, ed., 1984; Current Protocols in Molecular Biology (F. M. Ausubel et al., eds., 1994); PCR: The Polymerase Chain Reaction (Mullis et al., eds., 1994); Gene Transfer and Expression: A Laboratory Manual (Kriegler, 1990), and Fairfield, D. 1994. Chapter 10, Pelleting Cost Center. In Feed Manufacturing Technology IV. (McEllhiney, editor), American Feed Industry Association, Arlington, Va., pp. 110-139.

Unless defined otherwise herein, all technical and scientific terms used herein have the same meaning as commonly understood by one of ordinary skill in the art to which the present teachings belong. Singleton, et al., Dictionary of Microbiology and Molecular Biology, second ed., John Wiley and Sons, New York (1994), and Hale & Markham, The Harper Collins Dictionary of Biology, Harper Perennial, NY (1991) provide one of skill with a general dictionary of many of the terms used in this invention. Any methods and materials similar or equivalent to those described herein can be used in the practice or testing of the present teachings.

Numeric ranges provided herein are inclusive of the numbers defining the range.

### Definitions

As used herein, the term "granule" refers to a particle which contains a core, an active agent, and optionally at least one coating layer.

As used herein, the term "core" refers to the inner nucleus of a granule. The cores of the present teachings may be produced by a variety of fabrication techniques including: rotary atomization, wet granulation, dry granulation, spray drying, disc granulation, extrusion, pan coating, spheronization, drum granulation, fluid-bed agglomeration, high-shear granulation, fluid-bed spray coating, crystallization, precipitation, emulsion gelation, spinning disc atomization and other casting approaches, and prill processes. Such processes are known in the art and are described in US Pat. No. 4689297 and US Pat. No. 5324649 (fluid bed processing); EP656058B1 and US Pat. No. 454332 (extrusion process); US Pat. No. 6248706 (granulation, high-shear); and EP804532B1 and US Pat. No. 6534466 (combination processes utilizing a fluid bed core and mixer coating). The sandwich granule of the present teachings comprises a core upon which at least three coating layers are built.

The core includes the active agent, which may or may not be coated around a seed. Suitable cores for use in the present teachings are preferably a hydratable or porous material (i.e., a material which is dispersible or soluble in water) that is a feed grade material. The core material can either disperse in water (disintegrate when hydrated) or solubilize in water by going into a true aqueous solution. Clays (for example, the phyllosilicates bentonite, kaolin, montmorillonite, hectorite, saponite, beidellite, attapulgite, and stevensite), silicates, such as sand (sodium silicate), nonpareils and agglomerated potato starch or flour, or other starch granule sources such as wheat and corn cobs are considered dispersible. (Nonpareils are spherical particles made of a seed crystal that has been built onto and rounded into a spherical shape by binding layers of powder and solute to the seed crystal in a rotating spherical container. Nonpareils are typically made from a combination of a sugar such as sucrose, and a powder such as cornstarch.) In one embodiment of the present teachings the core comprises a sodium chloride or sodium sulfate crystal, also referred to as a seed, or other inorganic salt crystal. In another embodiment of the present teachings, the core comprises a sucrose crystal seed. Particles composed of inorganic salts and/or sugars and/or small organic molecules may be used as the cores of the present teachings. Suitable water soluble ingredients for incorporation into cores include: inorganic salts such as sodium chloride, ammonium sulfate, sodium sulfate, magnesium sulfate, zinc sulfate; or urea, citric acid, sugars such as sucrose, lactose and the like. Cores of the present teachings may further comprise one or more of the following: additional active agents, feed or food grade polymers, fillers, plasticizers, fibrous materials, extenders and other compounds known to be used in cores. Suitable polymers include polyvinyl alcohol (PVA), including partially and fully hydrolyzed PVA, polyethylene glycol, polyethylene oxide, polyvinyl pyrrolidine, and carbohydrate polymers (such as starch, amylose, amylopectin, alpha and beta-glucans, pectin, glycogen), including mixtures and derivatives thereof. Suitable fillers useful in the cores include inert materials used to add bulk and reduce cost, or used for the purpose of adjusting the intended enzyme activity in the finished granule. Examples of such fillers include, but are not limited to, water soluble agents such as salts, sugars and water dispersible agents such as clays, talc, silicates, cellulose and starches, and cellulose and starch derivatives. Suitable plasticizers useful in the cores of the present teachings are low molecular weight organic compounds and are highly specific to the polymer being plasticized. Examples include, but are not limited to, sugars (such as, glucose, fructose and sucrose), sugar alcohols (such as, sorbitol, xylitol and maltitol and other glycols), polar low molecular weight organic compounds, such as urea, or other known plasticizers such as water or feed grade plasticizers. Suitable fibrous materials useful in the cores of the present teachings include, but are not limited to: cellulose, and cellulose derivatives such as HPMC (hydroxy-propyl-methyl cellulose), CMC (carboxy-methyl cellulose), HEC (hydroxy-ethyl cellulose). In one embodiment, particularly for feed applications, of the present teachings, the core comprises a water-soluble or dispersible corn cob material or sugar or salt crystal. In another embodiment particularly suitable for household cleaning applications, the core comprises a water-soluble or dispersible sugar or salt crystal or a non pareil. Those skilled in the art will recognize that, for feed and food applications, the cores (and any polymers, fillers, plasticizers, fibrous materials, and extenders), are acceptable for food and/or feed applications. For household cleaning applications, such a restriction need not apply.

The terms "coating layer" and "layer" are used interchangeably herein. The first coating layer generally encapsulates the core in order to form a substantially continuous layer so that the core surface has few or no uncoated areas. Subsequent coating layers can encapsulate the growing granule to form one or more additional substantially continuous layer(s). The materials (e.g. the active agents and components detailed herein) used in the granule and/or multi-layered granule are suitable for the use in foods and/or animal feeds, and accordingly can be food grade or feed grade.

The term "outer coating layer" as used herein refers to the coating layer of a multi-layered granule which is the furthest from the core (i.e. the last coating layer which is applied). In some embodiments, the outer coating layer is the second moisture barrier layer.

The term "first moisture barrier layer" as used herein refers to a layer that surrounds the core of a sandwich granule, and which comprises at least one moisture barrier material.

The term "moisture hydrating layer" as used herein refers to a layer that surrounds the first moisture barrier layer of a sandwich granule, and which comprises at least one moisture hydrating material.

The term "second moisture barrier layer" as used herein refers to a layer that surrounds the moisture hydrating layer of a sandwich granule, and which comprises at least one moisture barrier material.

The term "moisture barrier material" refers to materials that exclude, prevent or substantially retard water uptake. These materials typically are hydrophobic or amphiphilic, provide insulation against water and do not inherently absorb and/or bind water and include, but are not limited to, film-forming materials. Examples of moisture barrier materials include barrier polymers, proteins, lipids, fats and oils, fatty acids and gums. Examples of film forming moisture barrier materials are natural and modified barrier polymers, such as gum arabic, whey, whey protein concentrate, PVA, including modified PVA and fully hydrolyzed PVA, and synthetic polymers such as latex, HPMC, and acid-thinned hydroxypropyl starch, for example, PureCoteTM, oxidized starch, and modified starch. Non-film forming moisture barrier materials include, for instance, waxes, fats, oils and lipids, and lecithin. Selected moisture barrier materials that do not readily oxidize are, for example, latex polymer and barrier polymers such as gum arabic.

The term "moisture hydrating material" refers to materials that take up aqueous liquids, such as water, by one several mechanisms. In a first non-limiting mechanism, the materials absorb free water. In a second non-limiting mechanism, the materials take up bound water that generally is present as crystalline waters of hydration. Accordingly, the materials may be provided as partially or fully hydrated materials or as non-hydrated materials that will absorb or bind aqueous liquids and retard or reduce the rate or extent of migration of such liquids to the active agent. In a third non-limiting mechanism, moisture hydrating materials thermally insulate the active agent by retarding heat transfer to the active agent within the granule and by maintaining the active agent at a lower temperature than the temperature at the exterior surface of the granule. Moisture hydrating materials include carbohydrates and inorganic salts, including hydrated salts, such as magnesium sulfate, sodium sulfate, and ammonium sulfate; maltodextrin; sugars, for example, sucrose; starch, including cornstarch.

As used herein, the terms "pellets" and "pelleting" refer to solid, rounded, spherical and cylindrical tablets or pellets and the processes for forming such solid shapes, particularly feed pellets and solid, extruded animal feed. Known food and animal feed pelleting manufacturing processes generally include admixing together food or feed ingredients for about 1 to about 5 minutes at room temperature, transferring the resulting admixture to a surge bin, conveying the admixture to a steam conditioner, optionally transferring the steam conditioned admixture to an expander, transferring the admixture to the pellet mill or extruder, and finally transferring the pellets into a pellet cooler. Fairfield, D. 1994. Chapter 10, Pelleting Cost Center. In Feed Manufacturing Technology IV. (McEllhiney, editor), American Feed Industry Association, Arlington, Va., pp. 110-139.

As used herein, the term "unpelleted mixtures" refers to premixes or precursors, base mixes, mash, and diluents. Premixes typically contain vitamins and trace minerals. Base mixes typically contain food and feed ingredients such as dicalcium phosphate, limestone, salt and a vitamin and mineral premix, but not grains and protein ingredients. Diluents include, but are not limited to grains (for example wheat middlings and rice bran) and clays, such as phyllosilicates (the magnesium silicate sepiolite, bentonite, kaolin, montmorillonite, hectorite, saponite, beidellite, attapulgite, and stevensite). Clays also function as carriers and fluidizing agent, or diluents, for feed premixes. Mash typically comprises a complete animal diet.

As used herein, the term "recovered activity" refers to the ratio of (i) the activity of an active agent after a treatment involving one or more of the following stressors: heating, increased pressure, increased pH, decreased pH, storage, drying, exposure to surfactant(s), exposure to solvent(s) (including water/moisture), and mechanical stress) to (ii) the activity of the phytase before the treatment. The recovered activity may be expressed as a percentage.

The percent recovered activity is calculated as follows:

$$\% \text{ recovered activity} = \left| \frac{(\text{ activity after treatment })}{(\text{ activity before treatment })} \right| \times 100\,\%$$

In the context of pelleting experiments, the "activity before treatment" can be approximated by measuring the active agent activity present in the mash that does not undergo treatment in a manner that is otherwise matched to the active agent that does undergo treatment. For example, the active agent in the untreated mash is handled and stored for a similar time and under similar conditions as the active agent in the treated (e.g. pelleted) mash, to control for possible interactions or other effects outside of the specified treatment per se.

As used herein, the term "active agent" comprises an enzyme.

Any enzyme may be used, and a nonlimiting list of enzymes include phytases, xylanases, 3-glucanases, phosphatases, proteases, amylases (alpha or beta or glucoamylases) cellulases, lipases, cutinases, oxidases, transferases, reductases, hemicellulases, mannanases, esterases, isomerases, pectinases, lactases, peroxidases, laccases, other redox enzymes and mixtures thereof. Particularly preferred enzymes include a xylanase from *Trichoderma reesei* and a variant xylanase from *Trichoderma reesei*, both available from DuPont Industrial Biosciences or the inherently thermostable xylanase described in EP1222256B1, as well as other xylanases from *Aspergillus niger, Aspergillus kawachii, Aspergillus tubigensis, Bacillus circulans, Bacillus pumilus, Bacillus subtilis, Neocallimastix patriciarum,Penicillium species, Streptomyces lividans, Streptomyces thermoviolaceus, Thermomonospora fusca, Trichoderma harzianum, Trichoderma reesei, Trichoderma viride.* Additional particularly preferred enzymes include phytases, such as for example Finase L®, a phytase from Aspergillus sp., available from AB Enzymes, Darmstadt, Germany; Phyzyme™ XP, a phytase from E. Coli, available from DuPont Nutrition and Health, and other phytases from, for example, the following organisms: Trichoderma, Penicillium, Fusarium, Buttiauxella, Citrobacter, Enterobacter, Penicillium, Humicola, Bacillus, and Peniophora, as well as those phytases described in US patent applications 61/595,923 and 61/595,941, both filed February 12, 2012 . An example of a cellullase is Multifect® BGL, a cellulase (beta glucanase), available from DuPont Industrial Biosciences and other cellulases from species such as Aspergillus, Trichoderma, Penicillium, Humicola, Bacillus, Cellulomonas, Penicillium, Thermomonospore, Clostridium, and Hypocrea. The cellulases and endoglucanases described in US20060193897A1 also may be used. Amylases may be, for example, from species such as Aspergillus, Trichoderma, Penicillium, Bacillus, for instance, *B. subtilis, B. stearothermophilus, B. lentus, B. licheniformis, B. coagulans,* and *B. amyloliquefaciens.* Suitable fungal amylases are derived from *Aspergillus*, such as *A. oryzae* and *A. niger.* Proteases may be from *Bacillus amyloliquefaciens*, *Bacillus lentus, Bacillus subtilis, Bacillus licheniformis, and Aspergillus and Trichoderma species.* Phytases, xylanases, phosphatases, proteases, amylases, esterases, redox enzymes, lipases, transferases, cellulases, and β- glucanases are enzymes frequently used for inclusion in animal feed. Enzymes suitable for inclusion into tablets for household care applications are similar, particularly proteases, amylases, lipases, hemicellulases, redox enzymes, peroxidases, transferases, and cellulases. In particularly preferred aspects of the present teachings, the enzymes are selected from phytases, xylanases, beta glucanases, amylases, proteases, lipases, esterases, and mixtures thereof. In one embodiment of the present invention, two enzymes are provided in the granule, a xylanase and a beta-glucanase. The enzymes may be mixed together or applied to the granule separately. In another embodiment, three enzymes are provided in the granule, namely beta-glucanase, xylanase and phytase. The above enzyme lists are examples only and are not meant to be exclusive. Any enzyme may be used in the sandwich granules of the present invention, including wild type, recombinant and variant enzymes of bacterial, fungal, yeast, plant, insect and animal sources, and acid, neutral or alkaline enzymes. It will be recognized by those skilled in the art that the amount of enzyme used will depend, at least in part, upon the type and property of the selected enzyme and the intended use.

**Exemplary Embodiments**

In an embodiment illustrative of the invention according to Figure 1, a granule comprises a seed (such as a salt crystal, for example a sodium sulfate crystal), around which an active agent such as an enzyme is coated. The resulting core can then be subjected to a fluid-bed spray coating process for addition of the various layers to make a sandwich granule. As depicted here, a first moisture barrier layer is present, followed by a moisture hydrating layer, which in turn is followed by a second moisture barrier layer.

In some embodiments, the seed and enzyme are made using fluid-bed spray coating, such that the enzyme is deposited as a coating onto a seed, to make a core. In some embodiments, the seed and enzyme are made through other means, such that the enzyme does not comprise a layer over the seed but can rather be interspersed with any of a variety of material(s).

In some embodiments, the first moisture barrier layer is directly adjacent to the core, such that there are no intervening layers. In some embodiments, the moisture hydrating layer is directly adjacent to the first moisture barrier layer, such that there are no intervening layers. In some embodiments, the second moisture barrier layer is directly adjacent to the moisture hydrating layer, such that there are no intervening layers. In some embodiments, there are no intervening layers between the core, the first moisture barrier layer, the moisture hydrating layer, and the second moisture barrier layer.

In one embodiment, the entire granule is made using fluid-bed spray coating, wherein a seed is first coated with an enzyme layer, the enzyme layer is next coated with a first moisture barrier layer, then a moisture hydrating layer is added, and finally a second moisture barrier layer is added. In such a granule, no intervening layers between the layers are implemented. However, in some embodiments, one or more additional intervening layers can be added.

In some embodiments, the first moisture barrier layer and the second moisture barrier layer comprise the same moisture barrier material or materials. As set out in the claims, the moisture barrier material of the first moisture barrier layer comprises PVA and talc, wherein the PVA is fully hydrolysed.

In some embodiments, the first moisture barrier layer comprises a single moisture barrier material. In some embodiments, the first moisture barrier layer comprises at least 2, 3, or 4 moisture barrier materials.

In some embodiments, the second moisture barrier layer comprises a single moisture barrier material. In some embodiments, the second moisture barrier layer comprises at least 2, 3, or 4 moisture barrier materials.

In some embodiments, the moisture hydrating layer comprises a single moisture hydrating material. In some embodiments, the moisture hydrating layer comprises at least 2, 3, or 4 moisture hydrating materials.

In some embodiments, the first moisture barrier layer and the second moisture barrier layer each individually comprise 9% w/w of the granule. In some embodiments, the first moisture barrier layer and the second moisture barrier each individually comprise 8-10%, 7-11%, 6-12%, 5-13%, 4-13%, 3-14%, 2-15%, or 1-16% w/w of the granule. In some embodiments, the moisture hydrating layer comprises 40% w/w of the granule. In some embodiments, the moisture hydrating layer comprises 39-41%, 38- 42%, 37-43%, 36-44%, 35-45%, 34-46%, 33-47%, 32-48%, or 31-49% w/w of the granule.

As will be appreciated by one of skill in the art, as the w/w % of the moisture barrier layers increases, the w/w % of the moisture hydrating layer can correspondingly decrease, and vice versa. Thus, in some embodiments, the first moisture barrier layer and the second moisture barrier layer each individually comprise 8-10%, 7-11%, or 6- 12% w/w of the granule, and the moisture hydrating layer correspondingly comprises 39-41%, 38-42%, or 37-43% w/w of the granule. It will also be appreciated by one of skill in the art that increasing or decreasing the w/w of the first and second moisture barrier layers can be performed without changing the w/w of the moisture hydrating layer, and vice versa, and other intervening layers, or the core, can change in their w/w to constitute the entire 100% of the granule.

In some embodiments, the moisture hydrating layer comprises PVA and starch. In some embodiments, the PVA is fully hydrolyzed.

In some embodiments, the sandwich granules of the present teachings comprise an active agent that retains at least 60, 65, 70, 75, 80, 85, 90, or 95% activity after a steam-heated pelleting process conducted between 85-95C for 5 minutes, where the sandwich granule is an ingredient.

In some embodiments, the sandwich granules of the present teachings comprise and active agent that retains at least 60, 65, 70, 75, 80, 85, 90, or 95% activity after storage of the granule in an unpelleted mixture comprising at least one compound selected from trace minerals, organic acids, reducing sugars, vitamins, choline chloride, and compounds which result in an acidic or a basic unpelleted mixture.

In some embodiments, the sandwich granule of the present teachings comprises an inorganic salt seed (for example sodium sulfate), an active agent including any of phytase, xylanase, B-glucanase, and amylase, a first moisture barrier layer comprising fully hydrolyzed PVA and talc (2-4% w/w fully hydrolyzed PVA and 5-7% talc), a moisture hydrating layer comprising starch and fully hydrolyzed PVA (for example 3-5% fully hydrolyzed PVA and 34-38% starch), and a second moisture barrier layer comprising fully hydrolyzed PVA and talc (for example 2-4% w/w fully hydrolyzed PVA and 5-7% talc). In some embodiments, the sandwich granule is made entirely by fluid-bed spray coating. In some embodiments, the sandwich granule is included in an animal feed pellet. In some embodiments, the sandwich granule is included in an animal feed unpelleted mixture. In some embodiments, the sandwich granule is included in a process for making an animal feed composition. In some embodiments, the sandwich granule is used in a steam-treating or pelleting process.

The invention can be further understood by reference to the following example, which is provided by way of illustration and not meant to be limiting.

**EXAMPLE:**

In this example, a collection of different granules were made with varying coating layers employing conventional fluid-bed spray coating procedures. The components of these granules are depicted in Table 1. More specifically, the PVA is either partially hydrolyzed (PVA PH) or fully hydrolyzed (PVA FH), the seed is an anhydrous sodium sulfate crystal, and a phytase enzyme was employed as the active agent, present as part of the enzyme solids ("enz. sol.") which also include associated fermentation solids that are co-purified with the enzyme. Subsequent to the formation of the different granules, the granules were exposed to a steam treatment process, and the resulting phytase activity of the granules was measured.

These data illustrate that Granule 2 possesses superior phytase activity following the steam treatment process as compared to the other granules. Granule 2 comprises a sandwiched structure, wherein a first moisture barrier layer ("SP2") and a second moisture barrier layer (SP4) flank a moisture hydrating layer (SP3). Surprisingly, Granule 2 had superior activity compared to a Granule 3, even though Granule 3 contained the same overall amount of moisture barrier materials (here, PVA and talc) as did Granule 2, illustrating the unexpected benefit provided by the sandwich granule structure. Further, comparing Granule 2 to Granule 4 illustrates the impact that fully hydrolyzed PVA (PVA FH) has relative to partially hydrolyzed PVA (PVA PH) when present in the moisture barrier layers as the moisture barrier material.

Similar results were obtained for other enzymes, including amylase and xylanase.

Table 1

| Granule | Core | Coating Layers | | | | | | | | | Pelleting Recovery 90C |
| | | SP1 | % | SP2 | % | SP3 | % | SP4 | % | |
|---|---|---|---|---|---|---|---|---|---|---|
| 1 | Sod. Sulf. | Enz. Sol. PVA PH Starch | 2.9 1 5 | PVA FH Starch | 4 36 | PVA FH Talc | 3 6 | | | 34% |
| 2 | Sod. Sulf. | Enz. Sol. PVA PH Starch | 2.9 1 5 | PVA FH Talc | 3 6 | PVA FH Starch | 4 36 | PVA FH Talc | 3 6 | 80% |
| 3 | Sod. Sulf. | Enz. Sol. PVA PH Starch | 2.9 1 5 | PVA FH Talc | 6 12 | PVA FH Starch | 4 36 | | | 61% |
| 4 | Sod. Sulf. | Enz. Sol. PVA PH Starch | 2.9 1 5 | PVA PH Talc | 3 6 | PVA PH Starch | 4 36 | PVA PH Talc | 3 6 | 20% |
| 5 | Sod. Sulf. | Enz. Sol. PVA PH Starch | 2.9 1 5 | | | | | | | |

**Claims**

1.  A granule comprising;
    a core comprising an active agent that comprises an enzyme;
    a first moisture barrier layer comprising a moisture barrier material;
    a moisture hydrating layer comprising a moisture hydrating material surrounding the first moisture barrier layer; and,
    a second moisture barrier layer comprising a moisture barrier material surrounding the moisture hydrating layer;
    wherein the moisture barrier material of the first moisture barrier layer comprises PVA and talc, wherein the PVA is fully hydrolysed and present at 2%-4% w/w of the granule, and the talc is present at 5%-7% w/w of the granule.

2.  The granule according to claim 1, wherein the core comprises a seed comprising a salt crystal.

3.  The granule according to any of the preceding claims, wherein the moisture barrier material is selected from barrier polymers, proteins, lipids, fats and oils, fatty acids, and gums.

4.  The granule according to any of the preceding claims, wherein the moisture hydrating material is selected from starch, inorganic salts, and sugar.

5.  The granule according to any of the preceding claims, wherein the moisture hydrating material of the moisture hydrating layer comprises PVA and starch, and optionally wherein the PVA is fully hydrolysed, and present at 2%-6% w/w of the granule, and wherein the starch is present at 34%-38% w/w of the granule.

6.  The granule according to any of the preceding claims, wherein the moisture barrier material of the second moisture barrier layer comprises PVA and talc, and optionally wherein the PVA is fully hydrolysed, and present at 2%-4% w/w of the granule, and wherein in the talc is present at 5%-7% w/w of the granule.

7.  The granule according to any of claims 2 to 6, wherein the seed comprises sodium sulfate.

8.  The granule according to any one of the preceding claims, wherein the enzyme is a phytase.

9.  The granule according to any of the preceding claims wherein the core further comprises PVA and starch, and optionally wherein the PVA is partially hydrolyzed PVA and is present at .75%-1.25% w/w of the granule, and wherein the starch is present at 4%-6% w/w of the granule.

10. The granule according to any of the preceding claims, wherein no intervening layers exist between the core, the first moisture barrier layer, the moisture hydrating layer, and the second moisture barrier layer.

11. An animal feed pellet comprising the granule of any of the preceding claims.

12. An animal feed unpelleted mixture comprising the granule of any of claims 1 to 10.

13. A process for producing an animal feed composition comprising:

    preparing granules according to any one of claims 1 to 10, the granules having a core comprising an active agent, a first moisture barrier layer, a moisture hydrating layer, and second moisture barrier layer;
    mixing the granules together with an unpelleted mixture; and,
    pelleting the unpelleted mixture at a temperature of 70°C-95°C.

14. The use of a granule according to any one of claims 1 to 10 in a steam-treating or pelleting process.

**Patentansprüche**

1.  Granulat, umfassend:

    einen Kern, umfassend einen Wirkstoff, der ein Enzym umfasst;
    eine erste Feuchtigkeitssperrschicht, die ein Feuchtigkeitssperrmaterial umfasst;
    eine feuchtigkeitsspendende Schicht, die ein feuchtigkeitsspendendes Material umfasst, das die erste Feuchtigkeitssperrschicht umgibt; und

eine zweite Feuchtigkeitssperrschicht, die ein Feuchtigkeitssperrmaterial umfasst, das die feuchtigkeitsspendende Schicht umgibt;

wobei das Feuchtigkeitssperrmaterial der ersten Feuchtigkeitssperrschicht PVA und Talkum umfasst, wobei das PVA vollständig hydrolysiert ist und mit 2% bis 4 Gew.-% des Granulats vorliegt und das Talkum mit 5% bis 7 Gew.-% des Granulats vorliegt.

2. Granulat nach Anspruch 1, wobei der Kern einen Salzkristall umfassenden Impfkristall umfasst.

3. Granulat nach einem der vorhergehenden Ansprüche, wobei das Feuchtigkeitssperrmaterial ausgewählt ist aus Sperrschicht-Polymeren, Proteinen, Lipiden, Fetten und Ölen, Fettsäuren und Gummen.

4. Granulat nach einem der vorhergehenden Ansprüche, wobei das feuchtigkeitsspendende Material ausgewählt ist aus Stärke, anorganischen Salzen und Zucker.

5. Granulat nach einem der vorhergehenden Ansprüche, wobei das feuchtigkeitsspendende Material der feuchtigkeitsspendenden Schicht PVA und Stärke umfasst und wobei gegebenenfalls das PVA vollständig hydrolysiert ist und mit 2% bis 6 Gew.-% des Granulats vorliegt und wobei die Stärke mit 34% bis 38 Gew.-% des Granulats vorliegt.

6. Granulat nach einem der vorhergehenden Ansprüche, wobei das feuchtigkeitsspendende Material der zweiten Feuchtigkeitssperrschicht PVA und Talkum umfasst und wobei gegebenenfalls das PVA vollständig hydrolysiert ist und mit 2% bis 4 Gew.-% des Granulats vorliegt und wobei das Talkum mit 5% bis 7 Gew.-% des Granulats vorliegt.

7. Granulat nach einem der Ansprüche 2 bis 6, wobei der Impfkristall Natriumsulfat umfasst.

8. Granulat nach einem der vorhergehenden Ansprüche, wobei das Enzym eine Phytase ist.

9. Granulat nach einem der vorhergehenden Ansprüche, wobei der Kern ferner PVA und Stärke umfasst und wobei gegebenenfalls das PVA teilweise hydrolysiertes PVA ist und mit 0,75 % bis 1,25 Gew.-% des Granulats vorliegt und wobei die Stärke mit 4% bis 6 Gew.-% des Granulats vorliegt.

10. Granulat nach einem der vorhergehenden Ansprüche, wobei zwischen dem Kern, der ersten Feuchtigkeitssperrschicht, der feuchtigkeitsspendenden Schicht und der zweiten Feuchtigkeitssperrschicht keine dazwischenliegenden Schichten vorhanden sind.

11. Tierfutterpellet, umfassend das Granulat nach einem der vorhergehenden Ansprüche.

12. Nicht pelletierte Tierfuttermischung, umfassend das Granulat nach einem der Ansprüche 1 bis 10.

13. Verfahren zum Herstellen einer Tierfutterzusammensetzung, umfassend:

Herstellen von Granulaten nach einem der Ansprüche 1 bis 10, wobei die Granulate einen Kern aufweisen, umfassend einen Wirkstoff, eine erste Feuchtigkeitssperrschicht, eine feuchtigkeitsspendende Schicht und eine zweite Feuchtigkeitssperrschicht;

Mischen der Granulate mit einer nicht pelletisierten Mischung; und

Pelletisieren der nicht pelletisierten Mischung bei einer Temperatur von 70° bis 95 °C.

14. Verwenden eines Granulats nach einem der Ansprüche 1 bis 10 in einem Dampf behandelnden oder pelletisierenden Prozess.

**Revendications**

1. Granulé comprenant:

un cœur comprenant un agent actif qui comprend une enzyme;

une première couche barrière à l'humidité comprenant un matériau barrière à l'humidité;

une couche d'hydratation absorbant l'eau comprenant une matière hydratante absorbant l'eau entourant la première couche barrière à l'humidité; et

une seconde couche barrière à l'humidité comprenant un matériau barrière à l'humidité entourant la couche d'hydratation absorbant l'eau;

dans lequel le matériau barrière à l'humidité de la première couche barrière à l'humidité comprend du PVA et du talc, dans lequel le PVA est totalement hydrolysé et présent à 2% à 4% p/p du granulé, et le talc est présent à 5% à 7% p/p du granulé.

2. Granulé selon la revendication 1, dans lequel le cœur comprend un germe comprenant un cristal de sel.

3. Granulé selon l'une quelconque des revendications précédentes, dans lequel le matériau barrière à l'humidité est choisi parmi les polymères formant barrière, les protéines, les lipides, les matières grasses et les huiles, les acides gras et les gommes.

4. Granulé selon l'une quelconque des revendications précédentes, dans lequel la matière hydratante absorbant l'eau est choisie parmi un amidon, les sels inorganiques et un sucre.

5. Granulé selon l'une quelconque des revendications précédentes, dans lequel la matière hydratante absorbant l'eau de la couche d'hydratation absorbant l'eau comprend du PVA et un amidon, et facultativement dans lequel le PVA est totalement hydrolysé, et présent à 2% à 6% p/p du granulé, et dans lequel l'amidon est présent à 34% à 38% p/p du granulé.

6. Granulé selon l'une quelconque des revendications précédentes, dans lequel le matériau barrière à l'humidité de la seconde couche barrière à l'humidité comprend du PVA et du talc, et facultativement dans lequel le PVA est totalement hydrolysé, et présent à 2% à 4% p/p du granulé, et dans lequel le talc est présent à 5% 7% p/p du granulé.

7. Granulé selon l'une quelconque des revendications 2 à 6, dans lequel le germe comprend du sulfate de sodium.

8. Granulé selon l'une quelconque des revendications précédentes, dans lequel l'enzyme est une phytase.

9. Granulé selon l'une quelconque des revendications précédentes, dans lequel le cœur comprend en outre du PVA et un amidon, et facultativement dans lequel le PVA est un PVA partiellement hydrolysé et est présent à 0,75% à 1,25% p/p du granulé, et dans lequel l'amidon est présent à 4% à 6% p/p du granulé.

10. Granulé selon l'une quelconque des revendications précédentes, dans lequel il n'existe pas de couche intermédiaire entre le cœur, la première couche barrière à l'humidité, la couche d'hydratation absorbant l'eau et la seconde couche barrière à l'humidité.

11. Granulé d'alimentation pour animaux comprenant le granulé selon l'une quelconque des revendications précédentes.

12. Mélange d'alimentation pour animaux non granulé comprenant le granulé selon l'une quelconque des revendications 1 à 10.

13. Procédé pour produire une composition d'alimentation pour animaux, comprenant:

la préparation de granulés selon l'une quelconque des revendications 1 à 10, les granulés ayant un cœur comprenant un agent actif, une première couche barrière à l'humidité, une couche d'hydratation absorbant l'eau et une seconde couche barrière à l'humidité;

le mélange des granulés conjointement avec un mélange non granulé; et

la granulation du mélange non granulé à une température de 70°C à 95°C.

14. Utilisation d'un granulé selon l'une quelconque des revendications 1 à 10 dans un procédé de traitement à la vapeur ou de granulation.

Seed ⎫
       ⎬ Core
Enzyme ⎭

First Moisture Barrier Layer

Moisture Hydrating Layer

Second Moisture Barrier Layer

Sandwich Granule

## FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9854980 A
- WO 9739116 A
- WO 2007044968 A
- EP 1996028 A
- WO 9723606 A
- US 20080031998 A
- US 4689297 A
- US 5324649 A
- EP 656058 B1
- US 454332 A
- US 6248706 B
- EP 804532 B1
- US 6534466 B
- EP 1222256 B1
- US 61595923
- US 61595941 B
- US 20060193897 A1

### Non-patent literature cited in the description

- **KLIBANOV.** Stabilization of Enzymes against Thermal Inactivation. *Advances in Applied Microbiology,* 1983, vol. 29, 1-28
- **SAMBROOK et al.** Molecular Cloning: A Laboratory Manual. 1989
- Oligonucleotide Synthesis. 1984
- Current Protocols in Molecular Biology. 1994
- PCR: The Polymerase Chain Reaction. 1994
- Gene Transfer and Expression: A Laboratory Manual. Kriegler, 1990
- Pelleting Cost Center. **FAIRFIELD, D.** In Feed Manufacturing Technology IV. American Feed Industry Association, 1994, 110-139
- **SINGLETON et al.** Dictionary of Microbiology and Molecular Biology. John Wiley and Sons, 1994
- **HALE ; MARKHAM.** The Harper Collins Dictionary of Biology. Harper Perennial, 1991
- Pelleting Cost Center. **FAIRFIELD, D.** In Feed Manufacturing Technology IV. American Feed Industry Association, 1994, 110-139